# EUROPÄISCHE PATENTSCHRIFT

(11) **EP 0 577 622 B1**
(45) Veröffentlichungstag und Bekanntmachung des Hinweises auf die Patenterteilung: **18.12.1996**
(21) Anmeldenummer: 92905728.9
(22) Anmeldetag: 09.03.1992
(51) Int. Cl.: A61M 35/00

(54) **TRANSDERMALES THERAPEUTISCHES PFLASTER**
TRANSDERMAL THERAPEUTIC PLASTER
PANSEMENT THERAPEUTIQUE TRANSDERMIQUE

(30) Priorität: 27.03.1991 DE 4110027
(43) Veröffentlichungstag der Anmeldung: 12.01.1994
(73) Patentinhaber: LTS LOHMANN Therapie-Systeme GmbH, 56567 Neuwied (DE)
(72) Erfinder: ANHÄUSER, Dieter, D-5451 Melsbach (DE); DEURER, Lothar, D-5400 Koblenz 1 (DE); HILLE, Thomas, D-5450 Neuwied (DE); STEINBORN, Peter, 5450 Neuwied 22 (DE)
(74) Vertreter: Flaccus, Rolf-Dieter, Dr.
(86) Internationale Anmeldenummer: EP9200513
(87) Internationale Veröffentlichungsnummer: WO9217237

(56) Entgegenhaltungen:
- EP-A- 0 242 827
- EP-A- 0 335 231
- DE-A- 3 629 304
- US-A- 4 614 076

## Beschreibung

Die Erfindung betrifft ein Verfahren zur kontinuierlichen Herstellung transdermaler therapeutischer Pflaster mit einer Rückschicht, einer haftklebenden Wirkstoffreservoirschicht und einer wiederablösbaren Schutzschicht, wobei produktionsbedingter Wirkstoffverlust minimiert wird.

Transdermale therapeutische Pflaster (im weiteren TT-Pflaster) sind auf die Haut aufzubringende Arzneiformen mit dem Aussehen traditioneller Pflaster, die über die Haut abzugebende Arzneistoffe enthalten und als transdermale therapeutische Systeme bekannt sind. Ein therapeutisches System kann einen oder mehrere Arzneistoffe enthalten, die in vorausbestimmter Rate kontinuierlich über einen festgesetzten Zeitraum an einen festgelegten Anwendungsort abgegeben werden ("Heilmann, Klaus: Therapeutische Systeme Konzept und Realisation programmierter Arzneiverabreichung", 4. Auflage, Ferdinand Enke Verlag Stuttgart, 1984, S. 26).

Die therapeutische Nutzung derartiger Arzneiformen ist bekannt. Die Darreichungsforn sind meist aus mehreren Schichten aufgebaut und bestehen im einfachsten Falle aus einer Rückschicht, einem selbstklebenden Wirkstoffreservoir und einer vor der Applikation zu entfernenden Schutzschicht. Aus einleuchtenden Gründen sind für TT-Pflaster runde oder geometrische Formen mit abgerundeten Ecken das Design der Wahl. Zur Serienproduktion von TT-Pflastern geeignete Verfahren müssen einen einheitlichen Wirkstoffgehalt der einzelnen Pflaster gewährleisten. Sie sollen so einfach sein, daß die Produktion zu wirtschaftlich sinnvollen Kosten möglich ist, d.h. daß vor allem der Wirkstoffverlust gering gehalten werden muß.

Derartige Verfahren sind bekannt. Ein in der DE-PS 32 22 800 beschriebenes medizinisches Abgabesystem zur Verabreichung eines Wirkstoffs über die Haut besteht aus einer Unterlage, einem angrenzenden, einen Wirkstoff und eine Flüssigkeit, ein rheologisches Mittel wie Cellulose, ein Polysaccharid oder eine Siliciumverbindung enthaltendes Reservoir sowie eine an das Reservoir angrenzende Membran zur Bestimmung der Abgabegeschwindigkeit des Wirkstoffes aus dem System. Dieses System hat Nachteile. So ist die Einzeldosierung durch Aufstreichen einer flüssigen Zubereitung mit definierter Fläche und Dicke sehr schwierig, weshalb bei der Herstellung mit Wirkstoffverlust durch wegen abweichender Arzneistoffbeladung auszusondernde Einzelstücke zu rechnen ist. Außerdem besteht bei Beschädigung der Membran die Gefahr der schlagartigen Wirkstoffabgabe, was für den Patienten fatale Folgen hoben kann.

Das therapeutische System der DE-PS 36 29 304 besteht aus einem einen flüssigen Wirkstoff oder eine flüssige Wirkstoffzubereitung enthaltenden und einen oder mehrere allseitig von einer Matrix umgebene Hilfsstoffe mit Stütz- und Verteilungsfunktion aufweisenden Wirkstoffdepot mit einer abdeckenden Rückschicht, einer die Wirkstoffabgabe steuernden Matrix und einer haftklebenden Fixierungseinrichtung. Sie weist den Nachteil auf, daß nur flüssige Wirkstoffe oder Wirkstoffzubereitungen zugelassen sind, deren Dosierung so hohe Anforderungen stellt, daß eine wirkstoffverlustfreie Produktion nicht gewährleistet ist.

Bei einem durch die DE-PS 33 15 272 bekannten Verfahren werden zur Fertigung der TT-Pflaster wirkstoffhaltige Haftkleberschichten auf eine wirkstoffundurchlässige Schutzschicht aufgebracht und mit einer ebenfalls wirkstoffundurchlässigen Rückschicht abgedeckt. Beim Ausstanzen der Einzelpflaster werden alle Schichten außer der wiederablösbaren Schutzschicht durchtrennt. Durch den zwischen den Einzelpflastern stehenbleibenden Verschnitt kann der Wirkstoffverlust erheblich sein, wobei zu berücksichtigen ist, daß die in der transdermalen Therapie eingesetzten Wirkstoffe Sondermüll sind.

Der Erfindung liegt danach die Aufgabe zugrunde, ein kontinuierliches Verfahren zur Herstellung von TT-Pflastern bereitzustellen, bei dem Wirkstoffverlust möglichst weitgehend unterbunden werden und das technisch wenig aufwendig ist. Die Aufgabe wird durch das Verfahren nach Anspruch 1 gelöst. Bei diesem wird das Wirkstoffreservoir durch bekannte Beschichtungstechniken wie Beschichtung mit wirkstoffhaltigen Haftkleberlösungen, wäßrigen Haftkleberdispersionen oder aufgeschmolzenen Haftklebermassen hergestellt.

Das wirkstoffhaltige Reservoir aus wirkstoffhaltiger Haftklebeschicht und Rückschicht wird durch Quer- und Längsschneiden des Beschichtungsmaterials in Quadrate oder Rechtecke geschnitten. Die viereckigen Reservoire werden mit der durch den wirkstoffhaltigen Haftkleber gebildeten Schicht mittig in gewünschtem Abstand auf eine allseitig überstehende, wiederablösbare Schutzschicht geklebt. Anschließend wird die Schutzschicht mit einer mit wirkstofffreiem Haftkleber beschichteten Rückschicht abgedeckt, die deutlich breiter ist als die Wirkstoffreservoire, aber nicht so breit sein muß wie die Schutzschicht. Die Rückschicht des Reservoirs wird damit zur Sperrschicht, die verhindert, daß eine Wirkstoffmigration in die wirkstofffreie Haftkleberschicht erfolgt.

Das anschließende Ausstanzen erfolgt entlang außerhalb der Wirkstoffreservoire verlaufenden Konturen. Dabei wird die haftklebende Rückschicht durchtrennt, die wiederablösbare Schutzschicht bleibt unverletzt. Das zwischen den Einzelpflastern stehenbleibende Gitterwerk ist wirkstofffreier Abfall. Durch zur Laufrichtung senkrechtes Durchschneiden der Schutzschicht zwischen den Reservoiren entstehen die erfindungsgemäßen TT-Pflaster.

Das erfindungsgemäße TT-Pflaster ist mehrschichtig aufgebaut. Die Rückschicht kann aus flexiblem oder nicht flexiblem Material bestehen. Zu ihrer Herstellung können Polymerfolien oder Metallfolien, etwa Aluminiumfolie allein oder mit einem polymeren Substrat beschichtet, verwandt werden. Es können auch textile Flächengebilde Verwendung finden, wenn sie undurchlässig für die Bestandteile der wirkstofffreien, ggf. aus einem Weich- oder Klebrigmacher bestehenden Haftkleberschicht sind. Bei einer bevorzugten Ausführungsform ist die Rückschicht eine mit Aluminium bedampfte Folie.

Die Haftkleberschicht besteht aus einer Polymermatrix mit einem Grundpolymer und gegebenenfalls den üblichen Zusätzen. Geeignete Polymere sind Silicone, Kautschuk. kautschukähnliche synthetische Homo-, Co- oder Blockpolymere, Polyacrylsäureester und deren Copolymere und Ester von hydriertem Kolophonium. Grundsätzlich kommen alle Polymere in Frage, die bei der Herstellung von Haftklebern eingesetzt werden und physiologisch unbedenklich sind. Besonders bevorzugt sind solche, die als Blockcopolymere auf Basis von Styrol und 1,3-Dienen, Polyisobutylenen oder Polymeren und Copolymeren aus Acrylat und/oder Methacrylat bestehen. Von den Blockcopolymeren auf Basis von Styrol und 1,3-Dienen finden bevorzugt lineare Styrol-Isopren-Styrol Blockcopolymere Verwendung.

Als Polymere auf Acrylatbasis sind beispielsweise Acrylatcopolymere aus 2-Ethylhexylacrylat, Vinylacetat und Acrylsäure mit bzw. ohne Titanchelatester bevorzugt. Als Ester von hydriertem Kolophonium werden vorzugsweise insbesondere dessen Methyl- und Glycerinester verwendet.

Die Art der als Zusätze möglichen Weichmacher und Klebrigmacher hängt vom verwendeten Polymer ab. Die in Frage kommenden physiologisch unbedenklichen Substanzen sind bekannt. Die Eigenklebrigkeit der Haftkleberschicht muß den dauernden Kontakt zur Haut allein sicherstellen. Sie kann im Hotmelt-Verfahren, als Lösung oder als Dispersionshaftkleber auf die Rückschicht aufgebracht werden.

Die Sperrschicht besteht vorzugsweise aus dem gleichen Material wie die Rückschicht. Sie soll während der Lagerung der Systeme die Diffusion des Wirkstoffes oder der Weichmacher aus dem Reservoir in die wirkstofffreie Haftkleberschicht verhindern.

Die Reservoirschicht besteht aus einer selbstklebenden Polymermatrix und dem Wirkstoff. Die Polymermatrix besteht aus einem Grundpolymer und gegebenenfalls den üblichen Zusätzen. Die Auswahl des Grundpolymers richtet sich noch den chemischen und physikalischen Eigenschaften des Wirkstoffes. Die Polymere können aus der gleichen Gruppe wie die wirksfofffreie Kleberschicht ausgewählt werden.

Als Wirkstoffe werden Substanzen verwendet, die ohne oder mit Resorptionsvermittler auf der Haut appliziert eine lokale oder systemische Wirkung hervorrufen.

Stoffe mit lokaler Wirkung sind zum Beispiel Antitranspirantia, Fungizide, Bactericide und Bacteriostatica.

Stoffe mit systemischer Wirkung sind beispielsweise Antibiotika, Hormone, Antipyretica, Antidiabetica, Koronardilatatoren, herzwirksame Glycoside, Spasmolytica, Antihypertonica, Psychopharmaka, Migränemittel, Corticoide. Analgetica, Antikontrazeptiva, Antirheumatica, Anticholinergica, Sympatolytica, Sympatomimetica, Vasodilatatoren, Anticoagulantien und Antiarrhythmika.

Von dem verwandten Polymer und dem Wirkstoff abhängige mögliche Zusätze sind Weichmacher, Klebrigmacher, Stabilisatoren. Trägerstoffe. diffusions- und penetrationsregulierende Zusätze oder Füllstoffe. Die in Frage kommenden physiologisch unbedenklichen Substanzen sind bekannt. Die Eigenklebrigkeit der Reservoirschicht soll einen dauernden Kontakt zur Haut sicherstellen. Wie erwähnt, muß die Klebkraft der Reservoirschicht allein ausreichen, um die sichere Haftung der Reservoirschicht zu gewährleisten, da sie die Voraussetzung ist für eine ausreichende Wirkstoffabgabe des Systems. Sie kann durch den wirkstofffreien Haftkleberrand nicht kompensiert werden.

Die vor der Anwendung abzulösende Schutzschicht der Reservoirschicht kann beispielsweise aus denselben Materialien wie die zur Herstellung der Rückschicht verwendeten bestehen. Diese müssen jedoch - beispielsweise durch eine Siliconbehandlung - ablösbar gemacht werden. Andere ablösbare Schutzschichten sind beispielsweise Polytetrafluorethylen, behandeltes Papier, Cellophan, Polyvinylchlorid u. ä.

Die Erfindung wird anhand einer Zeichnung und eines Ausführungsbeispiels erläutert. In der Zeichnung zeigt:
Fig. 1 das erfindungsgemäße TT-Pflaster in Draufsicht;
Fig. 2 das TT-Pflaster der Fig. 1, geschnitten entlang II-II.

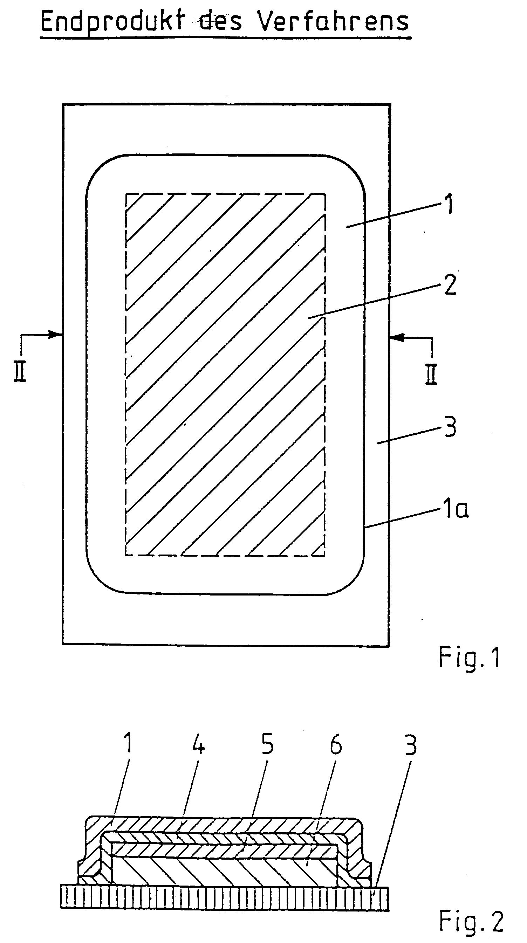

Fig. 1 zeigt schematisch ein erfindungsgemäßes TT-Pflaster in Draufsicht. Auf der wiederablösbaren hier rechteckigen Schutzschicht 3 liegt die mit wirkstofffreiem Haftkleber beschichtete Rückschicht 1 auf. Sie hat die Form eines Rechtecks mit abgerundeten Ecken. Andere Formen sind möglich. Die Stanzlinie 1a umreißt die Form der Rückschicht 1. Sie verläuft außerhalb des Laminats 2, um beim Ausstanzen Wirkstoffverlust zu vermeiden.

Innerhalb der Rückschicht 1 sind die Konturen des aus Reservoir 6 und Sperrschicht 5 bestehenden verdeckten rechteckigen Laminats 2 erkennbar. Die Rückschicht 1 überragt erfindungsgemäß das Laminat 2 allseitig. Es (2) hat die Form eines Vierecks, da es durch Querschneiden der zu einer Schmalrolle aufgewickelten Vorform entsteht. Durch die Wahl der Schnittrichtung bezogen auf die Laufrichtung kann bestimmt werden, ob beim Schneiden ein Rechteck, ein Quadrat, ein Parallelogramm oder ein Trapez entsteht. Die rechteckige Form wird bevorzugt. Zu beachten ist, daß beim Schneiden des Reservoirs nur bei der viereckigen Form des Laminats 2 Wirkstoffverluste wirklich vermeidbar sind.

Fig. 2 ist ein Querschnitt gemäß II - II der Fig. 1. Der Deutlichkeit wegen sind die Schichtdicken übertrieben dargestellt. Das Laminat 2 besteht aus dem Reservoir 6 und der Sperrschicht 5 und ruht auf der Schutzschicht 3, während die Sperrschicht 5 das Reservoir 6 von der die Rückschicht 1 bedeckenden wirkstofffreien Haftkleberschicht 4 trennt. Wie zu erkennen, wird das Laminat 2 von der Rückschicht 1 und der wirkstofffreien Haftkleberschicht 4 allseitig überragt.

### Beispiel:

Zur Herstellung des vorzugsweise in Schmalrollen vorgelegten Reservoirlaminats werden 3,45 kg einer 47,83 Gew-%igen Polyacrylatlösung eines selbstvemetzenden Acrylatcopolymers aus 2-Ethylhexylacrylat, Vinylacetat und Acrylsäure (Lösemittel : Ethylacetat : Heptan : Isopropanol : Toluol : Acetylaceton im Verhältnis von 37 : 26 : 26 : 4 : 1) mit 0,25 kg eines polyethylierten Glycerins mit C₈/C₁₀ Ethoxygruppen,
deren freie Hydroxylgruppen teilweise mit Capryl/Caprinsäuren verestert sind, 0,5 kg Methanol, 0,35 kg Glutarsäuremonomethylester, 0,125 kg eines Polymethacrylats auf Basis eines Copolymers aus Dimethylaminoethylmethacrylat und neutralen Methacrylsäureestern und 0,25 kg Buprenorpinbase eine Stunde lang innig vermischt.

Anschließend wird die Mischung so auf eine 420 mm breite transparente Polyesterfolie mit 50 µm Dicke aufgestrichen, daß das Flächengewicht der getrockneten Kleberschicht bei 120 g/m² liegt, Die durch Siliconbehandlung wieder ablösbare Polyesterfolie dient als Zwischenabdeckung. Nach dem Trocknen wird mit einer später als Sperrschicht dienenden wirkstoffundurchlässigen Polyesterfolie, Dicke 36 µm, Breite 420 mm, abgedeckt, Nach dem Schneiden des Laminats 2 in Längsrichtung in Abständen von 70 mm liegt das Laminat aus Zwischenabdeckung, Reservoirschicht und Sperrschicht in Schmalrollen vor.

Zur Herstellung der wirkstofffreien Kleberschicht werden
23,32 kg einer 47,83 Gew-%igen Polyacrylatlösung eines selbstvernetzenden Acrylatcopolymers aus 2-Ethylhexylacrylat, Vinylacetat- und Acrylsäure (Lösemittel : Ethylacetat : Heptan : Isopropanol : Toluol : Acetylaceton = 37 : 26 : 26 : 4 : 1) und 0,6 kg 2-Octyldodecanol
10 min lang verrührt und auf eine silikonierte transparente 100 µm dicke und 406 mm breite Polyesterfolie so aufgestrichen, daß das Flächengewicht der getrocketen Kleberschicht bei 120 g/m² liegt.

Nach dem Trocknen wird die Kleberschicht mit einer später als Rückschicht des Systems dienenden 23 µm dicken und 406 mm breiten hautfarbenen Polyesterfolie abgedeckt. Paralleles Schneiden in Längsrichtung in Abständen von 90 mm ergibt das wirkstofffreie Kleberlaminat aus Zwischenabdeckung, wirkstofffreier Kleberschicht und Rückschicht, ebenfalls in Schmalrollen.

Für die Schutzschicht werden aus einer silikonisierten, 100 µm Polyesterfolie Schmalrollen von 90 mm Breite hergerichtet.

Eine Schmalrolle des Reservoirlaminats aus Sperrschicht, Reservoirschicht und Zwischenabdeckung wird in eine mit einer geeigneten bekannten Vorrichtung versehene Abrollvorrichtung so eingespannt, daß die Zwischenabdeckung unten liegt. Unmittelbar unter der Vorrichtung läuft senkrecht zum Reservoirlaminat die 90 mm breite, 100 µm dicke als Schutzschicht vorgesehene Polyesterfolie mit der silikonierten Seite nach oben zu. Mit einer geeigneten Schneidevorrichtung werden Sperrschicht und Reservoirschicht des Reservoirlaminats, nicht jedoch die Zwischenabdeckung, in Abständen von 35,7 mm senkrecht zur Laufrichtung durchtrennt. Über die Spenderkante der bekannten Vorrichtung wird im spitzen Winkel die Zwischenabdeckung abgezogen. Auf die unmittelbar unter der Spenderkante laufende silikonierte Polyesterfolie werden die Rechtecke aus Reservoirschicht und Sperrschicht mit einer Kantenlänge von 70 mm x 35,7 mm mittig aufgeklebt. Da die silikonierte Polyesterfolie kontinuierlich läuft, die Rechtecke aber im Takt gespendet werden, ergibt sich ein Abstand zwischen den Rechtecken von 20 mm.

An einer zweiten Kaschierstation wird das in Schmalrollen vorliegende Laminat aus Rückschicht, wirkstofffreier Kleberschicht und Zwischenabdeckung so eingelegt, daß die Zwischenabdeckung unten liegt. Die Zwischenabdeckung wird maschinell abgezogen, das verbleibende Laminat aus Rückschicht und wirkstofffreier Haftkleberschicht kantengerade und parallel zur Laufrichtung von oben auf die auf der Schutzschicht sitzenden Rechtecke aufgelegt und das ganze aufgewickelt. Die erfindungsgemäßen TT-Pflaster liegen damit als Schmalrolle vor und sind nun noch zu vereinzeln.

Hierzu wird mit einem Stanzwerkzeug, das die Form eines Rechtecks mit abgerundeten Ecken und 90 mm x 50 mm Kantenlänge hat, die Rückschicht ohne die Schutzschicht so durchtrennt, daß das Rechteck aus Sperrschicht und Reservoirschicht zentrisch in der ausgestanzten Fläche liegt. Das wirkstofffreie zwischen den Systemen liegende Gitterwerk wird als Abfall abgezogen. Durch zum Bandverlauf senkrechtes Durchtrennen der Schutzschicht zwischen den einzelnen Systemen entstehen die erfindungsgemäßen TT-Pflaster, die anschließend verpackt werden.

## Patentansprüche

1. Verfahren zur kontinuierlichen Herstellung transdermaler therapeutischer Pflaster, die eine Rückschicht (1), eine haftklebende Wirkstoffreservoirschicht (6) und eine ablösbare Schutzschicht (3) aufweisen, wobei der Wirkstoffverlust während der Konfektionierung minimiert wird, und bei einem vorgelegten bandförmigen Laminat mit einer haftklebenden Rückschicht (1) und der ablösbaren Schutzschicht (3) zwischen die Schichten (1,3) in Längsrichtung hintereinander einzelne viereckige, haftklebende Wirkstoffreservoirabschnitte (2) eingelegt werden, **dadurch gekennzeichnet**, **daß** die Wirkstoffreservoirabschnitte (2) in Längsrichtung zwischen sich gleiche lichte Abstände aufweisen und deren Breite so bemessen wird, daß sie auf beiden Bandseiten von der Rückschicht (1) und der ablösbaren Schutzschicht (3) überragt werden und daß
zunächst die haftklebende Rückschicht (1) durch Stanzen so durchtrennt wird, daß die Stanzlinie (1a) rund um die einzelnen Wirkstoffreservoirabschnitte (2) mit deutlichem Abstand von deren Außenbegrenzungen verläuft,
daß der dabei entstehende gitterartige Verschnitt der wirkstofffreien haftklebenden Rückschicht (1) entfernt, und daß anschließend
in den entstandenen Zwischenräumen zwischen den Wirkstoffreservoirabschnitten (2) die Schutzschicht (3) durchtrennt wird.

2. Verfahren nach Anspruch 1,
**dadurch gekennzeichnet**, **daß** die haftklebende Wirkstoffreservoirschicht (2) vor dem Aufbringen auf die haftklebende Rückschicht (1) auf der zu dieser weisenden Seite durch eine wirkstoffundurchlässige Sperrschicht (5) abgedeckt wird.

## Claims

1. A process for the continuous production of transdermal therapeutic patches exhibiting a backing layer (1), a pressure-sensitive adhesive drug reservoir layer (6) and a removable protective layer (3), the loss of drug during fabrication being minimized, and, in a laminate which is present in a tape-like form and consists of a pressure-sensitive adhesive backing layer (1) and a removable protective layer (3), individual quadrangular pressure-sensitive adhesive drug reservoir sections (2) being inserted between the layers (1,3), lengthwise and one after the other, **characterized in that** the clearance between said drug reservoir sections (2) in longitudinal direction remains constant and the width thereof is dimensioned such that said backing layer (1) and said removable protective layer (3) project beyond the tape at both sides thereof, and that
the pressure-sensitive adhesive backing layer (1) is initially cut by punching in such a manner that the punching line (1a) surrounds the external dimensions of the individual drug reservoir sections (2) at a considerable distance therefrom,
that the resulting latticed refuse of the drug-free pressuresensitive adhesive backing layer (1) is removed,
and that the protective layer (3) is then cut in the resultant spaces between the drug reservoir sections (2).

2. The process according to claim 1, characterized in that, prior to applying the pressure-sensitive adhesive drug reservoir layer (2) to the pressure-sensitive adhesive backing layer (1), that side of said pressure-sensitive adhesive drug reservoir layer facing said backing layer is covered with a barrier layer (5) which is impermeable to active substances.

## Revendications

1. Procédé de fabrication continue de timbres thérapeutiques transdermiques qui présentent une couche dorsale (1), une couche-réservoir de principe actif autocollante (6) et une couche protectrice amovible (3), conformément auquel la perte de principe actif est minimisée au cours de la confection et conformément auquel, dans un lamifié en forme de bande présenté avec une couche dorsale autocollante (1) et la couche protectrice amovible (3) entre les couches (1, 3), on insère les unes après les autres, en direction longitudinale, des découpes (2) de réservoir de principe actif, individuelles, rectangulaires et autocollantes, caractérisé en ce que les découpes (2) de réservoir de principe actif présentent entre elles, en direction longitudinale, de faibles distances identiques et en ce que leurs largeurs sont mesurées en une manière telle qu'elles sont chevauchées des deux côtés de la bande par la couche dorsale (1) et la couche protectrice amovible (3) et en ce que la couche dorsale autocollante (1) est séparée par estampage en une manière telle que la ligne d'estampage (1a) se déroule de manière arrondie autour des découpes de réservoir de principe actif (2) à une nette distance de leurs limites externes, en ce que la découpe en forme de réseau qui prend ainsi naissance enlève la couche dorsale autocollante dépourvue de principe actif (1) et en ce que, ensuite, dans les espaces intermédiaires formés entre les découpes de réservoir de principe actif (2), la couche protectrice (3) soit découpée.

2. Procédé suivant la revendication 1, caractérisé en ce que la couche-réservoir de principe actif autocollante (2) est recouverte, avant l'application sur la couche dorsale autocollante (1) du côté qui se présente de cette dernière, par une couche-barrière imperméable au principe actif (5).
